(19) Europäisches Patentamt

European Patent Office

Office européen des brevets

(11) Veröffentlichungsnummer: **0 373 455 B1**

# (12) EUROPÄISCHE PATENTSCHRIFT

(45) Veröffentlichungstag der Patentschrift: **15.09.93**

(21) Anmeldenummer: **89122250.7**

(22) Anmeldetag: **02.12.89**

(51) Int. Cl.⁵: **A61M 1/34**

(54) Vorrichtung zur kontinuierlichen Hämofiltration und Hämodiafiltration.

(30) Priorität: **13.12.88 DE 3841863**

(43) Veröffentlichungstag der Anmeldung:
**20.06.90 Patentblatt 90/25**

(45) Bekanntmachung des Hinweises auf die
Patenterteilung:
**15.09.93 Patentblatt 93/37**

(84) Benannte Vertragsstaaten:
**BE DE ES FR GB IT NL SE**

(56) Entgegenhaltungen:
**EP-A- 0 212 127     EP-A- 0 222 709**
**DE-A- 2 607 022     DE-A- 2 634 238**
**DE-A- 2 838 414     DE-A- 3 111 061**
**DE-C- 3 637 771**

(73) Patentinhaber: **B. Braun Melsungen AG**
**Postfach 120**
**D-34209 Melsungen(DE)**

(72) Erfinder: **Rath, Dieter**
**Franz-Gleim-Strasse 69**
**D-3508 Melsungen(DE)**
Erfinder: **von der Haar, Friedrich, Prof. Dr. rer. nat.**
**Tilsiter Strasse 2**
**D-3508 Melsungen(DE)**

(74) Vertreter: **Selting, Günther, Dipl.-Ing. et al**
**Patentanwälte von Kreisler, Selting, Werner,**
**Postfach 10 22 41, Bahnhofsvorplatz 1**
**D-50462 Köln (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Vorrichtung der im Oberbegriff des Patentanspruchs 1 angegebenen Art.

Zur Entwässerung von Patienten auf Intensivstationen wird das Verfahren der kontinuierlichen arterio-venösen Hämofiltration (CAVH) oder der kontinuierlichen venovenösen Hamofiltration (CVVH) eingesetzt. Bei beiden Verfahren wird das Blut des Patienten über ein Filter geleitet, das dem Blut Wasser entzieht und zu dem Patienten wieder zurückführt. Bei der kontinuierlichen veno-venösen Hämofiltration wird zur Erreichung eines notwendigen Filtrationsdruckes eine Blutpumpe (Rollenpumpe) eingesetzt. Bei Mehrfach-Organversagen kommt häufig ein akutes Nierenversagen hinzu. Bei derartigen Patienten reicht die einfache CVVH (oder CAVH) nicht aus, um neben der Flüssigkeitsabfuhr auch eine genügende Elimination toxischer Substanzen zu erreichen. In solchen Fällen wurde bisher die Flüssigkeitsabfuhr durch Erhöhen des Innendruckes der Blutseite des Filters erhöht. Die erhöhte Filtrationsmenge wurde in einem Bilanzierungsgefäß gesammelt und daraus die Filtrationsrate bestimmt. Um den Mehrverlust an Flüssigkeit auszugleichen, wurde den Patienten über eine handelsübliche Infusionspumpe Substitutionslösung zugeführt. Die üblicherweise als Schlauchpumpen ausgeführten Infusionspumpen haben eine Genauigkeit von ca. 5 %, was bei länger dauerndem Betrieb zu hohen Bilanzierungsfehlern bei der Messung der zugeführten Flüssigkeit führt.

Auch die Bestimmung der den Patienten entnommenen Flüssigkeit ist in dem Bilanzierungsgefäß stark fehlerbehaftet.

Bekannt sind ferner Vorrichtungen zur kontinuierlichen veno-venösen Hämodiafiltration (CVVHD), die mit Doppelschlauchpumpen als Filtrations- und Substitutionspumpen für Dialysierflüssigkeit arbeiten, auch diese haben in der Regel eine zu ungenaue Mengenbilanzierung wegen der Toleranzabweichung der Pumpenschläuche. Wegen der hohen Fehlerrate kann es nach etwa fünf Tagen zu starker Ödembildung kommen.

Aus DE 36 37 771 A1 ist eine Infusionsvorrichtung bekannt, mit der es möglich ist, die Menge der dem Patienten zugeführten Flüssigkeit mit hoher Genauigkeit zu bestimmen. Die Infusionsflüssigkeit wird in eine volumetrische Meßkammer geleitet, deren Füllstand überwacht wird. Die Zeit, die erforderlich ist, um die Flüssigkeit mit einer Schlauchpumpe von einem oberen Füllstand bis zu einem unteren Füllstand der Meßkammer abzupumpen, wird gemessen und mit einer der Soll-Infusionsrate entsprechende Soll-Entleerungszeit in Beziehung gesetzt. Wenn die tatsächliche Entleerungszeit von der Soll-Entleerungszeit abweicht, wird die Pumpengeschwindigkeit entsprechend korrigiert, so daß der nächstfolgende Entleerungsvorgang in einer Zeit erfolgt, die der Soll-Entleerungszeit angenähert ist bzw. dieser entspricht. Diese bekannte Infusionsvorrichtung dient also dazu, eine vorgegebene Förderrate mit hoher Genauigkeit einzuhalten. Dies reicht jedoch für die Bilanzierung bei der Hämofiltration oder Hämodiafiltration nicht aus, weil sich dort die Flüssigkeitsmenge auf der Sekundärseite des Filters bzw. Dialysators selbständig einstellt und nicht durch eine Soll-Förderrate vorgegeben werden kann.

DE-A-31 11 061 und EP-A-0 222 709 beschreiben Blutreinigungsvorrichtungen, bei denen das Blut des Patienten mit einer Blutpumpe durch einen Blutfilter oder einen Dialysator gepumpt wird. Das Filtrat läuft in eine volumetrische Meßkammer, wobei durch die zum Füllen benötigte Zeit die Filtrationsrate gewonnen wird. An den Blutkreislauf ist ferner eine Substitutionsvorrichtung angeschlossen, aus der eine Substitutionslösung zugeführt wird. Die Substitutionsvorrichtung enthält eine volumetrische Meßkammer und eine Einrichtung zur Messung der von einer Substitutionspumpe zum Füllen oder Leeren dieser Meßkammer benötigten Zeit. Diese Vorrichtungen bilden jeweils ein voluminöses und komplexes Gerät, das zahlreiche Komponenten wie Pumpen, Meßkammer u.dgl. enthält und dessen Anschaffung und Anwendung einen Aufwand erfordern, der häufig über das für den jeweiligen Einsatz benötigte Maß hinaus geht.

Der Erfindung liegt die Aufgabe zugrunde, eine Vorrichtung zur kontinuierlichen Hämofiltration und Hämodiafiltration zu schaffen, die für unterschiedliche Anwendungsfälle variiert werden kann.

Die Lösung dieser Aufgabe erfolgt erfindungsgemäß mit den im Patentanspruch 1 angegebenen Merkmalen.

Die Erfindung ermöglicht es insbesondere, dem Patienten während der Hämofiltration oder Hämodiafiltration eine Substitutionslösung in einer Menge zuzuführen, die exakt derjenigen Flüssigkeitsmenge entspricht, die dem Körper entzogen und nicht wieder zugeführt wird. Hierbei werden die Förderraten sämtlicher Flüssigkeiten nach dem Meßkammerprinzip mit hoher Genauigkeit ermittelt, so daß die Substitutionsmenge ebenfalls mit hoher Genauigkeit errechnet werden kann.

Im folgenden werden unter Bezugnahme auf die Zeichnungen Ausführungsbeispiele der Erfindung näher erläutert.

Es zeigen:

Fig. 1    ein schematisches Diagramm der Vorrichtung zur kontinuierlichen Hämofiltration und Hämodialyse in Anwendung bei der Hämodiafiltration,

Fig. 2    die Vorrichtung nach Fig. 1, jedoch ohne Zuführung einer Substitutionslösung,

Fig. 3   die Vorrichtung bei der Benutzung für die Hämofiltration, mit Zufuhr von Substitutionslösung und

Fig. 4   ein abgewandeltes Ausführungsbeispiel bei der Benutzung für die Hämofiltration.

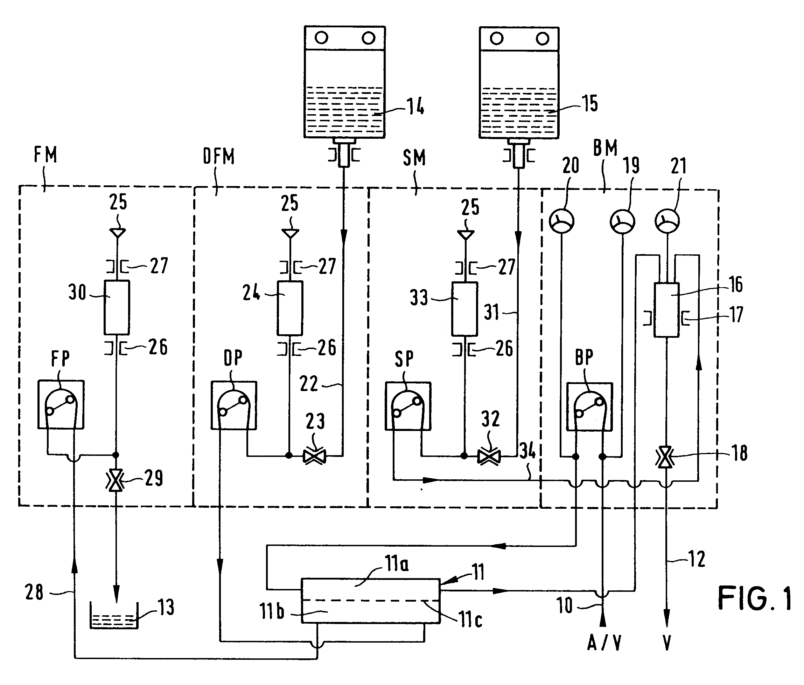

Die Vorrichtung nach Fig. 1 enthält generell vier Monitore, nämlich den Filtrationsmonitor FM, den Dialysierflüssigkeitsmonitor DFM, den Substitutionsmonitor SM und den Blutmonitor BM.

Der Blutmonitor BM dient dazu, dem Patienten über eine Leitung 10 Blut zu entziehen, dieses Blut durch einen Dialysator 11 oder ein Filter zu leiten und es anschließend über eine Leitung 12 dem Patienten wieder zuzuführen. Der Filtrationsmonitor FM ist an die Sekundärseite des Dialysators 11 oder Filters angeschlossen und er empfängt und mißt diejenige Flüssigkeitsmenge, die den Dialysator verläßt. Diese Flüssigkeit wird anschließend einem Ablauf 13 zugeführt. Der Dialysierflüssigkeitsmonitor DFM wird nur bei der Hämodiafiltration (Dialyse) benutzt. Er ist an einen Dialysierflüssigkeitsbehälter 14 angeschlossen und führt der Sekundärseite des Dialysators 11 in vorbestimmter Förderrate die Dialysierflüssigkeit zu. Der Substitutionsmonitor SM ist an einen Behälter 15 mit Substitutionslösung angeschlossen und er gibt an den Blutmonitor BM Substitutionslösung in solcher Menge ab, daß die dem Körper entzogene und nicht anderweitig zugeführte Flüssigkeitsmenge durch die Substitutionslösung ersetzt wird.

Der Blutmonitor BM enthält eine Blutpumpe BP, deren Einlaß an die vom Patienten kommende Leitung 10 angeschlossen ist und deren Auslaß mit dem Einlaß der Blutkammer 11a des Dialysators 11 verbunden ist. Die Blutkammer 11a ist von der Sekundärkammer 11b durch die Membran 11c getrennt. Der Auslaß der Blutkammer 11a ist mit einem Einlaß eines Pufferbehälters 16 verbunden, der einen Luftsensor 17 zur Ermittlung von eventuellen Luftblasen im Blut. Vom Pufferbehälter 16 führt die Leitung 12, die ein Ventil 18 enthält, zum Patienten. Diese Leitung 12 wird stets an eine Vene V des Patienten angeschlossen, während die Leitung 10 dann, wenn eine arterio-venöse Behandlung durchgeführt werden soll, an eine Arterie A angeschlossen wird, und, wenn eine veno-venöse Behandlung erfolgen soll, an eine Vene V angeschlossen wird.

Der Blutmonitor BM enthält außerdem verschiedene Druckmeßgeräte 19,20,21 zur Messung des Drucks vor und hinter der Blutpumpe BP sowie in dem druckdichten Puffergefäß 16.

Der Dialysierflüssigkeitsmonitor DFM enthält eine Leitung 22, die von dem Dialysierflüssigkeitsbehälter 14 über ein Ventil 23 zu der Meßkammer 24 und zum Einlaß der Dialysierflüssigkeitspumpe DP führt. Der Auslaß der Dialysierflüssigkeitspumpe DP ist an den Einlaß der Sekundärkammer 11b des Dialysators 11 angeschlossen. Das Ventil 23 wird jeweils über eine kurze Zeit geöffnet, so daß Dialysierflüssigkeit aus dem Behälter 14 unter Schwerkraftwirkung in die Meßkammer 24 einfließen kann. Die Pumpe DP kann hierbei weiterlaufen, weil ihre Förderrate viel geringer ist als die Rate der über das geöffnete Ventil 23 zufließenden Flüssigkeit. Das obere Ende der Meßkammer 24 ist über ein Belüftungsfilter 25 mit der Atmosphäre verbunden. Ferner weist die Meßkammer 24 einen unteren Füllstandsdetektor 26 und einen oberen Füllstandsdetektor 27 auf. Sobald nach dem Öffnen des Ventils 23 der obere Füllstandsdetektor 27 angezeigt hat, daß die Meßkammer 24 mit Dialysierflüssigkeit gefüllt ist, wird das Ventil 23 geschlossen. Die Pumpe DP entleert dann den Meßbehälter 24 mit konstanter Förderrate. Wenn der untere Füllstandsdetektor 26 angesprochen hat, wird die Zeit gemessen, die zum Entleeren der Meßkammer 24 benötigt wurde, also die Zeit zwischen dem Schließen des Ventils 23 und dem Ansprechen des unteren Füllstandsdetektors 26. Diese Zeit ist ein Maß für die tatsächliche Förderrate der Pumpe DP. Diese Förderrate wird einem (nicht dargestellten) Mikroprozessor zugeführt, der die Geschwindigkeit der Pumpe DP so einstellt, daß die Pumpe DP die Meßkammer in einer der Soll-Förderrate entsprechender Zeit entleert, so daß die Förderrate der Dialysierflüssigkeit gleich dem voreingestellten Sollwert wird.

Der Filtrationsmonitor FM enthält eine Filtrationspumpe FP, deren Einlaß über eine Leitung 28 mit dem Auslaß der Sekundärkammer 11b des Dialysators 11 verbunden ist. Der Auslaß der Filtrationspumpe FP ist über ein Ventil 29 mit dem Ablauf 13 oder mit einem Sammelbehälter verbunden. Ferner ist an den Auslaß der Pumpe FP das untere Ende einer Meßkammer 30 angeschlossen, in die bei geschlossenem Ventil 29 die Pumpe FP die Flüssigkeit hineinfördert. Auch die Meßkammer 30 ist mit einem Belüftungsfilter 25, einem unteren Füllstandsdetektor 26 und einem oberen Füllstandsdetektor 27 ausgestattet. Die Pumpe FP fördert die die Sekundärkammer 11b des Dialysators verlassende Flüssigkeit bei geschlossenem Ventil 29 in den Meßbehälter 30. Diejenige Zeit, die erforderlich ist, um den Flüssigkeitsstand der Meßkammer 30 vom unteren Füllstandsdetektor 26 bis zum oberen Füllstandsdetektor 27 zu erhöhen, wird von dem Mikroprozessor gemessen und dazu benutzt, die Laufgeschwindigkeit der Pumpe FP zu steuern. Wenn der obere Füllstandsdetektor 27 von dem oberen Füllstand der Meßkammer 30 aktiviert worden ist, wird das Ventil 29 geöffnet und die Flüssigkeit läuft aus der Meßkammer 30 schnell in den Ablauf 13 ab. Wenn der untere Füllstandsdetektor 26 vom unteren Füllstand aktiviert worden ist, wird das Ventil 29 wieder geschlossen, so daß die Pumpe FP die Meßkammer 30 von neuem füllt. Die Laufgeschwindigkeit der Pumpe FP wird nach jedem Entleerungsvorgang der Meßkammer 30 so korrigiert, daß die Förderrate der Pumpe FP exakt dem Flüssigkeitsanfall am Auslaß der Sekundärkammer 11b entspricht. Dies wird dadurch erreicht, daß die Laufgeschwindigkeit der Pumpe FP so eingestellt wird, daß sie der Füllzeit der Meßkammer 30 bei dem vorhergehenden Füllvorgang entspricht. Die Pumpe FP fördert also aus dem Dialysator 11 kontinuierlich eine Flüssigkeitsmenge, die exakt derjenigen Flüssigkeitsmenge entspricht, die aus der Sekundärkammer 11b abgeführt werden muß. Diese Flüssigkeitsmenge entspricht der Summe der der Sekundärkammer 11b von der Dialysierflüssigkeitspumpe DP zugeführten Flüssigkeit und dem Ultrafiltrat, das aus der Blutkammer 11a durch die Membran 11c hindurch in die Sekundärkammer 11b gelangt ist.

Der Substitutionsmonitor SM ist über eine Leitung 31 an einen Behälter 15 für Substitutionslösung anschließbar. Die Leitung 31 enthält ein Ventil 32, das gleichermaßen mit dem Meßbehälter 33 und dem Einlaß der Substitutionspumpe SP verbunden ist. Der Auslaß der Substitutionspumpe SP ist über eine Leitung 34 mit einem weiteren Einlaß des Pufferbehälters 16 des Blutmonitors BM verbunden.

Der Substitutionsmonitor SM liefert an den Blutmonitor BM eine Menge an Substitutionslösung mit einer bestimmten Förderrate. Diese Förderrate wird in der nachfolgend erläuterten Weise ermittelt. Die Überwachung der Förderrate erfolgt dadurch, daß die Laufgeschwindigkeit der Pumpe SP derart kontrolliert wird, daß diejenige Zeit, die erforderlich ist, damit der Flüssigkeitsstand in der Meßkammer 33 vom oberen Füllstandsdetektor 27 bis zum unteren Füllstandsdetektor 26 absinkt, einen Wert annimmt, der der vorgesehenen Förderrate entspricht. Zum Füllen der Meßkammer 33 wird das Ventil 32 geöffnet, so daß aus dem Behälter 15 Substitutionslösung unter Schwerkraftwirkung in den Meßbehälter 33 eingeführt wird. Bei Ansprechen des oberen Füllstandsdetektors 27 wird das Ventil 32 geschlossen und dann wird die Zeit gemessen, die zum Leeren der Meßkammer 33 benötigt wird. Die Geschwindigkeit der Pumpe SP wird danach so korrigiert, daß die Förderrate der Pumpe exakt dem vorgegebenen Wert entspricht.

Sämtliche verwendeten Pumpen FP,DP,SP und BP sind Schlauchpumpen, die beispielsweise als Rollenpumpen oder Taumelscheibenpumpen ausgebildet sind. Die Ventile 18,23,29 und 32 sind Schlauchventile, die die betreffende flexible Leitung abquetschen. Diese Ventile sind, ebenso wie die Pumpen von dem Mikroprozessor gesteuert.

Bei der Anwendung der Vorrichtung nach Fig. 1 zur kontinuierlichen arterio-venösen Hämodiafiltration (CAVHD) oder zur kontinuierlichen venovenösen Hämodiafiltration (CVVHD) wird die Blutpumpe BP mit einer vorgewählten Geschwindigkeit betrieben. Ferner wird die Dialysierflüssigkeitspumpe DP derart gesteuert, daß dem Einlaß der Sekundärkammer 11b ein konstanter Fluß mit vorgewählter Flüssigkeitsrate (Volumen pro Zeiteinheit) zugeführt wird. Durch die Membran 11 gelangen Blutbestardteile von der Blutkammer 11a in die Sekundärkammer 11b, so daß die Menge der Flüssigkeit in der Sekundärkammer 11b sich erhöht. Das Filtrationsvolumen FV, das dem Filtrationsmonitor von der Sekundärkammer 11b angeboten wird, ist also größer als das Dialysierflüssigkeitsvolumen DV, das der Sekundärkammer 11b von dem Dialysierflüssigkeitsmonitor DFM zugeführt wird.

Das Substitutionsvolumen SV, das dem Blutmonitor BM von dem Substitutionsmonitor SM zugeführt werden muß, um eine ausgeglichene Mengenbilanz zu erhalten, errechnet zu

$$SV = FV - DV$$

oder bei zusätzlich gewünschter Ultrafiltration zu

$$SV = FV - DV - UFV,$$

wobei UFV die Ultrafiltrationsrate ist.

Die Größe SV wird von dem Mikroprozessor errechnet und die Geschwindigkeit der Substitutionspumpe SP wird so eingestellt, daß diese Pumpe das errechnete Substitutionsvolumen SV pro Zeiteinheit fördert. Durch die Überwachung der Entleerungszeit der Meßkammer 33 wird die Einhaltung dieser Förderrate kontrolliert.

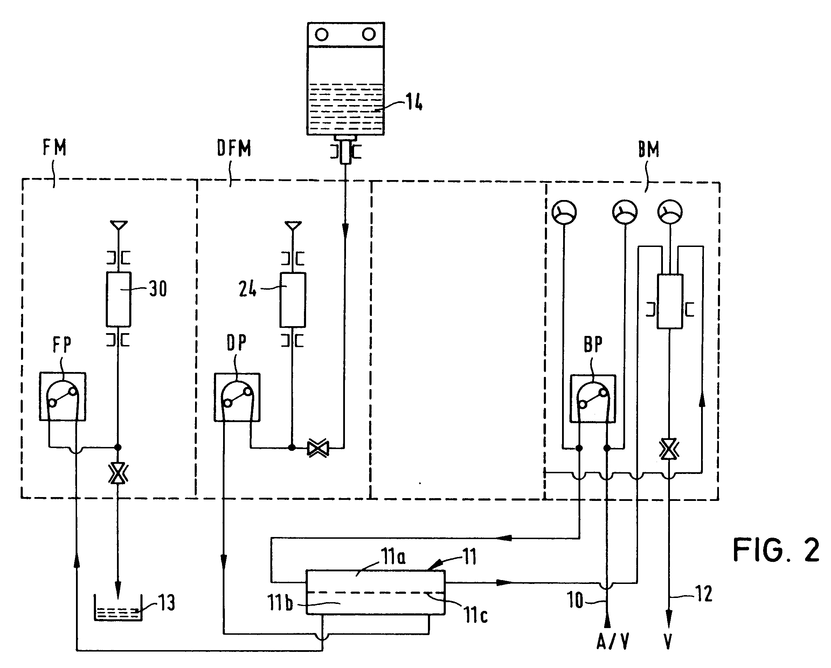

Fig. 2 zeigt die Vorrichtung nach Fig. 1 bei ihrer Verwendung ohne Substitutionslösung, wobei der Substitutionsmonitor SM fortgelassen ist. Das dem Dialysator 11 zugeführte Dialysierflüssigkeitsvolumen DV wird entsprechend einer vorgewählten Rate von der Pumpe DP gefördert und das die Sekundärkammer 11b des Dialysators verlassende Filtrationsvolumen FV wird von dem Filtrationsmonitor FM in der oben beschriebenen Weise gemessen. Das Ultrafiltrationsvolumen UFV, also dasjenige Volumen, das die Dialysatormembran 11c pro Zeiteinheit passiert, wird als Ultrafiltrationsrate errechnet zu

$$UFV = FV - DV.$$

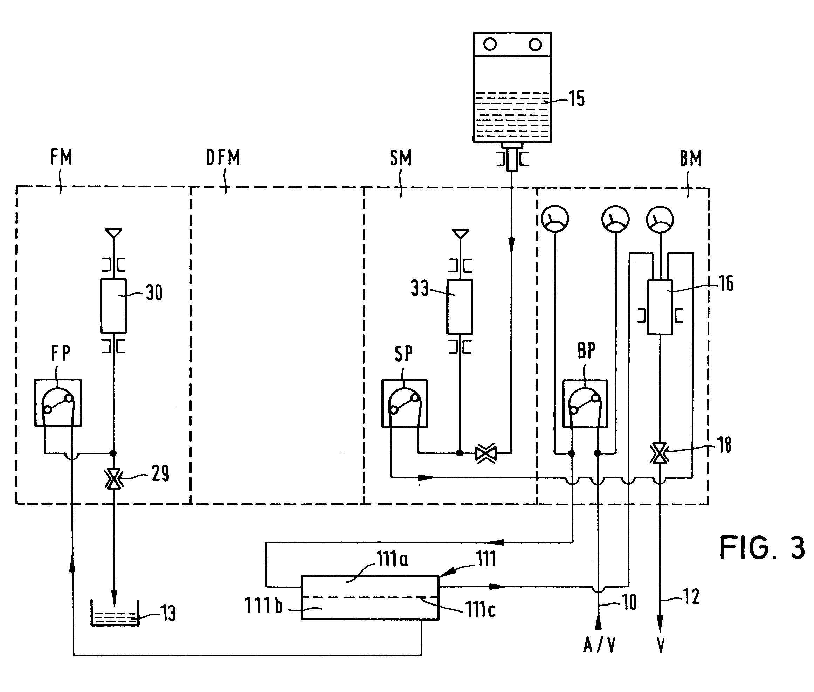

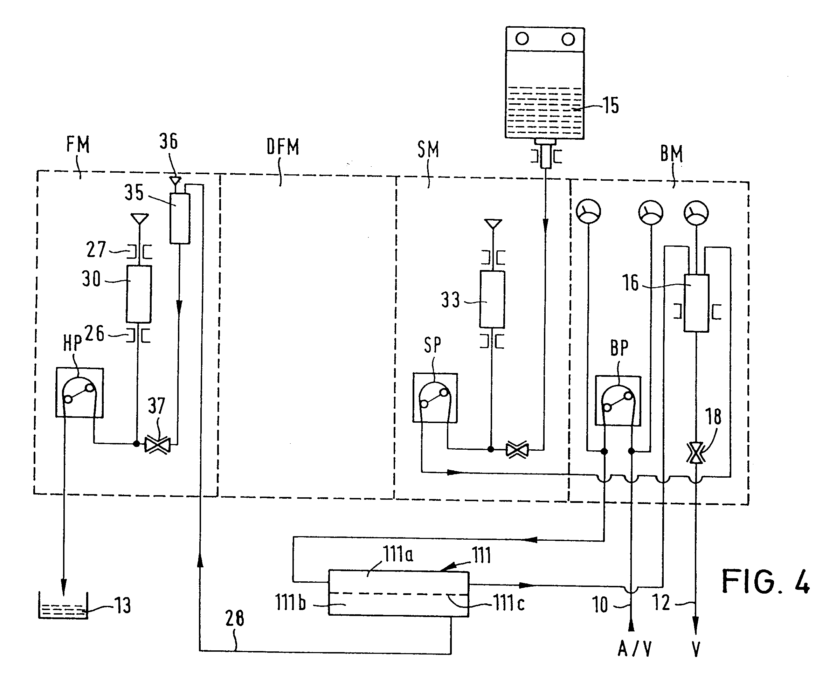

Bei dem Ausführungsbeispiel von Fig. 1 wird die Vorrichtung in Verbindung mit einem Dialysator oder Hämofilter für die CAVHD oder CVVHD benutzt. Dieselbe Vorrichtung kann auch in Verbindung mit einem Blutfilter für die kontinuierliche arterio-venöse Hämofiltration (CAVH) oder die kontinuierliche veno-venöse Hämofiltration (CVVH) benutzt werden. Bei diesen Filtrationen wird der Dialysator 11 durch ein Blutfilter 111 ersetzt, bei dem die Sekundärkammer 111b nur einen einzigen Anschluß als Auslaß aufweist. Ein Teil des im Blut enthaltenen Wassers passiert die Filtermembran 111c und gelangt in die Sekundärkammer 111b des Blutfilters, von wo es durch den Filtrationsmonitor FM abgeführt wird, so wie dies in den Fign. 3 und 4 dargestellt ist.

Fig. 4 zeigt ein Ausführungsbeispiel der Vorrichtung für die CAVH oder CVVH, wobei ebenfalls der Dialysierflüssigkeitsmonitor DFM nicht vorhanden ist. Der Unterschied gegenüber dem ersten Ausführungsbeispiel besteht in dem abweichenden Aufbau des Filtrationsmonitors FM. Gemäß Fig. 4 ist die an die Sekundärkammer 111b des Blutfilters 111 angeschlossene Leitung 28 zu einer Meßkammer 30 geführt, die tiefer angeordnet ist als das Blutfilter 111, so daß das Filtrat unter Schwerkrafteinfluß in die Meßkammer 30 einfließen kann. Bei geöffnetem Ventil 37 wird diejenige Zeit gemessen, die zum Füllen der Meßkammer 30 erforderlich ist. Bei Erreichen des oberen Sensors 27 wird das Ventil 37 geschlossen und die weiter anfallende Filtratmenge in einem Pufferbehälter 35 mit Belüftungsventil 36 zwischengespeichert und der Inhalt der Meßkammer 30 über die Hilfspumpe HP mit maximaler Fördergeschwindigkeit entleert. Bei Erreichen des unteren Sensors 26 wird die Pumpe gestoppt und Ventil 37 wieder geöffnet. Es steigt erneut Filtrat in die Meßkammer, bis der obere Sensor 27 wieder erreicht wird. Aus der Zeitspanne zwischen dem 1. und dem 2. Erreichen des Sensors 27, bzw. dem 2. und 3. Erreichen usw. kann die jeweilige angefallene Filtratmenge bestimmt werden. Der Filtrationsmonitor FM von Fig. 4 kann natürlich auch in Verbindung mit einem Dialysator 11 verwendet werden.

Bei den Ausführungsformen von Fig. 3 und 4 wird die Förderrate SV der Substitutionspumpe SP so gesteuert, daß sie gleich der vom Filtrationsmonitor FM festgestellten Lieferrate FV der Sekundärkammer 111b des Filters 111 ist:

$$SV = FV$$

oder bei zusätzlich gewünschter Ultrafiltration

$$SV = FV - UFV.$$

Anstelle der Hilfspumpe HP kann auch ein Ventil vorgesehen werden und Meßkammer 30 entleert sich durch Schwerkraft.

Die erfindungsgemäße Vorrichtung erlaubt die Entwässerung des Blutes eines Patienten und zusätzlich bei Nierenversagen eine Dialysebehandlung, wobei lediglich das Blutfilter (111) durch den Dialysator (11) ersetzt werden muß. Blutfilter und Dialysator sind leicht gegeneinander austauschbar bzw. auswechselbar.

## Patentansprüche

1. Vorrichtung zur kontinuierlichen Hämofiltration und Hämodiafiltration, mit
   einer Blutpumpe (BP),
   einem in Reihe mit der Blutpumpe (BP) an den Blutkreislauf des Patienten anschließbaren Blutfilter (111),
   einem das Filtrat aus dem Blutfilter ableitenden Filtrationsmonitor (FM), der eine volumetrische Meßkammer (30) und eine Einrichtung zur Messung der zum Füllen der Meßkammer (30) benötigten Zeit, und somit zur Messung des Anfalls von Filtrationsflüssigkeit, aufweist, wobei die Rate der Filtrationsflüssigkeit für eine Mengenbilanzierung der dem Patienten entnommenen und zugeführten Flüssigkeit ausgewertet wird, und
   einem Substitutionsmonitor (SM), der einen Behälter (15) mit Substitutionslösung, eine Substitutionspumpe (SP), eine volumetrische Meßkammer (33) und eine Einrichtung zur Messung der von der Substitutionspumpe (SP) zum Füllen oder Leeren dieser Meßkammer (33) benötigten Zeit, und somit zur Messung der Förderrate der Substitutionspumpe (SP), aufweist, wobei die Förderrate der Substitutionspumpe (SP) in Abhängigkeit von der gemessenen Füll- oder Entleerungszeit der Meßkammer (33) korrigiert wird, **dadurch gekennzeichnet,** daß der Substitutionsmonitor (SM) ein einzeln zustellbares Gerät ist, dessen Behälter (15) zur Abgabe von Substitutionslösung mit einem Blutmonitor (BM) verbunden ist, der auch die Blutpumpe (BP) enthält, daß das Blutfilter (111) gegen einen Dialysator (11) austauschbar ist, und
   daß als weiteres, einzeln zustellbares Gerät ein Dialysierflüssigkeitsmonitor (DFM) vorgesehen ist, der einen Behälter (14) mit Dialysierflüssigkeit mit dem Dialysator (11) verbindet und eine Dialysierflüssigkeitspumpe (DP), eine volumetrische Meßkammer (24) und eine Einrichtung zur Messung der von der Dialysierflüssigkeitspumpe (DP) zum Füllen oder Leeren dieser Meßkammer (24) benötigten Zeit, und somit zur Messung der Förderrate der Dialysierflüssigkeitspumpe (DP), aufweist und die Förderrate der Dialysierflüssigkeitspumpe (DP) entsprechend der Füll- oder Entleerungszeit der Meßkammer (24) auf einen vorgegebenen Wert korrigiert.

**2.** Vorrichtung nach Anspruch 1, dadurch gekennzeichnet, daß der Substitutionsmonitor (SM) und der Dialysierflüssigkeitsmonitor (DFM) von gleichem Aufbau sind.

**3.** Vorrichtung nach Anspruch 1 oder 2, dadurch gekennzeichnet, daß der Filtrationsmonitor (FM) von gleichem Aufbau ist wie der Substitutionsmonitor (SM) oder der Dialysierflüssigkeitsmonitor (DFM) und zusätzlich eine gegenüber der Meßkammer (30) erhöht angeordnete Pufferkammer (35) zur Aufnahme von der Meßkammer (30) zuzuführender Filtrierflüssigkeit aufweist, und daß eine Hilfspumpe (HP) oder ein Ventil die Flüssigkeit aus der Meßkammer (30) in einen Ablauf (13) leitet.

**4.** Vorrichtung nach einem der Ansprüche 1 bis 3, dadurch gekennzeichnet, daß der Filtrationsmonitor (FM) eine Flüssigkeit aus dem Filter (111) oder Dialysator (11) ansaugende Filtrationspumpe (FP) enthält, die die Flüssigkeit in den Meßbehälter (30) fördert, daß diese Flüssigkeit im Anschluß an das Füllen des Meßbehälters (30) über eine Ventilvorrichtung (29) aus dem Meßbehälter (30) einem Ablauf (13) zugeführt wird, und daß die Förderrate der Filtrationspumpe (FP) in Abhängigkeit von der zum Füllen des Meßbehälters benötigten Zeit so verändert wird, daß die Entleerungszeit gleich der Füllzeit wird.

**5.** Vorrichtung nach einem der Ansprüche 1 bis 4, dadurch gekennzeichnet, daß die Meßkammer (30) des Filtrationsmonitors (FM) an ihrem unteren Ende mit einer Einlaßleitung (28) und über ein Ventil (38) mit dem Ablauf (13) verbunden ist, wobei bei geschlossenem Ventil (38) die Füllzeit der Meßkammer (30) gemessen wird.

**6.** Vorrichtung nach einem der vorhergehenden Ansprüche, dadurch gekennzeichnet, daß bei der Hämodiafiltration die Förderrate (SV) der Substitutionspumpe (SP) derart gesteuert ist, daß sie der Differenz (FV - DV) der Förderraten von Filtrationsmonitor (FM) und Dialysierflüssigkeitsmonitor (DFM) entspricht.

**7.** Vorrichtung nach einem der Ansprüche 1 bis 5, dadurch gekennzeichnet, daß bei der Hämofiltration die Förderrate (SV) der Substitutionspumpe (SP) derart gesteuert ist, daß sie der Förderrate (FV) des Filtrationsmonitors (FM) entspricht.

**Claims**

**1.** A device for the continuous hemofiltration and hemodiafiltration, comprising
a blood pump (BP),
a blood filter (111) being connectable in series with said blood pump (BP) to the blood circuit of the patient,
a filtration monitor (FM) conducting the filtrate out of said blood filter and comprising a volumetric measuring chamber (30) and means for measuring the time required for filling said measuring chamber (30), and thus for measuring the volume of filtration liquid, the rate of the filtration liquid being evaluated for a quantity balancing of the liquid withdrawn from and supplied to the patient, and
a substitution monitor (SM) comprising a container (15) with substitution solution, a substitution pump (SP), a volumetric measuring chamber (33) and means for measuring the time said substitution pump (SP) needs to fill or empty said measuring chamber (33), and thus for measuring the feed rate of said substitution pump (SP), the feed rate of said substitution pump (SP) being corrected depending on the measured filling or emptying time of said measuring chamber (33),
**characterized in**
that said substitution monitor (SM) is an individually addable apparatus, the container (15) of which, in order to supply substitution solution, is connected to a blood monitor (BM) which also comprises said blood pump (BP),
that said blood filter (111) is exchangeable for a dialyser (11), and
that a dialysis liquid monitor (DFM) is provided as a further individually addable apparatus, which connects a container (14) containing dialysis liquid to the dialyser (11) and comprises a dialysis liquid pump (DP), a volumetric measuring chamber (24), and means for measuring the time the dialysis liquid pump (DP) needs to fill or empty said measuring chamber (24), and thus for measuring the feed rate of said dialysis liquid pump (DP), and which corrects the feed rate of said dialysis liquid pump (DP) corresponding to the filling or emptying time of said measuring chamber (24) to a predetermined value.

**2.** A device according to claim 1, characterized in that said substitution monitor (SM) and said dialysis liquid monitor (DFM) are of the same structure.

**3.** A device according to claim 1 or 2, characterized in that said filtration monitor (FM) is of the same structure as said substitution monitor (SM) or said dialysis liquid monitor (DFM), and additionally comprises a buffer chamber (35) for receiving filtering liquid to be supplied to said measuring chamber (30), which buffer chamber is arranged at an elevated position with respect to said measuring chamber (30), and that an auxiliary pump (HP) or a valve leads the liquid from said measuring chamber (30) into an outlet (13).

4. A device according to one of claims 1 to 3, characterized in that said filtration monitor (FM) comprises a filtration pump (FP) sucking liquid off said filter (111) or said dialyser (11) and feeding this liquid into said measuring container (30), that, subsequent to the filling of said measuring container (30), this liquid is fed from said measuring container (30) to an outlet (13) via a valve means (29), and that the feed rate of said filtration pump (FP) is changed depending on the time required for filling said measuring container such that the emptying time becomes equal to the filling time.

5. A device according to one of claims 1 to 4, characterized in that said measuring chamber (30) of said filtration monitor (FM) is connected, at its lower end, to an inlet conduit (28) and via a valve (38) to said outlet (13), the filling time of said measuring chamber (30) being measured when said valve (38) is closed.

6. A device according to one of the preceding claims, characterized in that, in case of hemodiafiltration, the feed rate (SV) of said substitution pump (SP) is controlled such that it corresponds to the difference (FV - DV) of the feed rates of the filtration monitor (FM) and the dialysis liquid monitor (DFM).

7. A device according to one of claims 1 to 5, characterized in that, in case of hemofiltration, the feed rate (SV) of said substitution pump (SP) is controlled such that it corresponds to the feed rate (FV) of said filtration monitor (FM).

**Revendications**

1. Dispositif de filtrage de sang en continu et de filtrage d'hémodialyse en continu comprenant :
   une pompe à sang (BP),
   un filtre à sang (111) qui peut être raccordé en série avec la pompe à sang (BP) à la circulation sanguine du patient,
   un dispositif de surveillance (FM) de filtrage, dérivant le filtrat hors du filtre à sang, qui comprend une chambre de mesure volumétrique (30) et une installation de mesure du temps nécessaire pour le remplissage de la chambre de mesure (30) et, de cette manière, de mesure de la production de liquide de filtrage, le débit du liquide de filtrage étant évalué en vue de l'établissement d'un bilan quantitatif du liquide prélevé dans le patient et ramené vers lui, et

un dispositif de surveillance de substitution (SM), qui comprend un récipient (15) recevant une solution de substitution, une pompe de substitution (SP), une chambre de mesure volumétrique (33) et une installation de mesure du temps nécessaire à la pompe de substitution (SP) pour remplir ou vider cette chambre de mesure (33) et, de cette manière, de mesure du débit de refoulement de la pompe de substitution (SP), le débit de refoulement de la pompe de substitution (SP) étant corrigé en fonction du temps mesuré de remplissage ou de vidage de la chambre de mesure (33),

   **caractérisé en ce que**

le dispositif de surveillance (SM) de substitution est un appareil adjoingnable isolément, dont le récipient (15) est relié, pour la délivrance de solution de substitution, à un dispositif de surveillance de sang (BM), qui contient également la pompe à sang (BP),

en ce que le filtre à sang (111) peut être échangé contre un dialyseur (11), et

en ce qu'il est prévu comme autre appareil adjoingnable isolément un dispositif de surveillance de liquide de dialyse (DFM), qui relie au dialyseur (11) un récipient (14) de liquide de dialyse et qui comprend une pompe (DP) à liquide de dialyse, une chambre de mesure volumétrique (24) et une installation de mesure du temps nécessaire à la pompe (DP) à liquide de dialyse pour remplir ou vider cette chambre de mesure (24) et, de cette manière, de mesure du débit de refoulement de la pompe (DP) à liquide de dialyse, et qui corrige à une valeur prédéterminée le débit de refoulement de la pompe (DP) à liquide de dialyse en fonction du temps de remplissage ou de vidage de la chambre de mesure (24).

2. Dispositif selon la revendication 1, caractérisé en ce que le dispositif de surveillance (SM) de substitution et le dispositif de surveillance (DFM) de liquide de dialyse sont de même structure.

3. Dispositif selon la revendication 1 ou 2, caractérisé en ce que le dispositif de surveillance (FM) de filtrage est de même structure que le dispositif de surveillance (SM) de substitution ou le dispositif de surveillance (DFM) de liquide de dialyse et comprend en outre une chambre tampon (35), surélevée par rapport à la chambre de mesure (30), pour recevoir le liquide de filtrage à amener à la chambre de mesure (30) et en ce qu'une pompe auxiliaire (HP) ou une vanne conduit le liquide hors de la chambre de mesure (30) dans une décharge (13).

4. Dispositif selon l'une des revendications 1 à 3, caractérisé en ce que le dispositif de surveillance (FM) de filtrage contient une pompe (FP) de filtrage, aspirant un liquide hors du filtre (111) ou du dialyseur (11), qui refoule le liquide dans le récipient de mesure (30), en ce que ce liquide est amené, à la suite du remplissage du récipient de mesure (30), hors du récipient (30) par un dispositif de vanne (29) à une décharge (13), et en ce que le débit de refoulement de la pompe (FP) de filtrage est modifié, en fonction du temps nécessaire pour le remplissage du récipient de mesure, de manière que le temps de vidage soit égal au temps de remplissage.

5. Dispositif selon l'une des revendications 1 à 4, caractérisé en ce que la chambre de mesure (30) du dispositif de surveillance (FM) de filtrage est reliée à une conduite d'admission (28) à son extrémité inférieure, et à la décharge (13) par une vanne (38), le temps de remplissage de la chambre de mesure (30) étant mesuré lorsque la vanne (38) est fermée.

6. Dispositif selon l'une des revendications précédentes, caractérisé en ce que le débit de refoulement (SV) de la pompe (SP) de substitution est commandé, lors du filtrage d'hémodialyse, d'une manière telle qu'il correspond à la différence (FV - DV) des débits de refoulement du dispositif de surveillance (FM) de filtrage et du dispositif de surveillance (DFM) de liquide de dialyse.

7. Dispositif selon l'une des revendications 1 à 5, caractérisé en ce que le débit de refoulement (SV) de la pompe (SP) de substitution est commandé, lors du filtrage de sang, d'une manière telle qu'il correspond au débit de refoulement (FV) du dispositif de surveillance (FM) de filtrage.

FIG. 1

FIG. 2

FIG. 3

FIG. 4

EP 0 373 455 B1